(19) Europäisches Patentamt
European Patent Office
Office européen des brevets

(11) **EP 3 998 586 A1**

(12) **EUROPEAN PATENT APPLICATION**

(43) Date of publication:
**18.05.2022  Bulletin 2022/20**

(21) Application number: **21207007.2**

(22) Date of filing: **08.11.2021**

(51) International Patent Classification (IPC):
**G06V 20/69** (2022.01)      **G06V 10/82** (2022.01)
**G06V 10/74** (2022.01)      **G06K 9/62** (2022.01)

(52) Cooperative Patent Classification (CPC):
**G06V 20/698; G06K 9/628;** G06V 10/761;
G06V 10/82; G06V 2201/03

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **09.11.2020  KR 20200148939**

(71) Applicant: **Lunit Inc.
Seoul 06241 (KR)**

(72) Inventor: **LEE, Jeong Hoon
06241 Seoul (KR)**

(74) Representative: **Bongiovanni, Simone et al
Studio Torta S.p.A.
Via Viotti, 9
10121 Torino (IT)**

(54) **METHOD AND SYSTEM FOR PREDICTING RESPONSIVENESS TO THERAPY FOR CANCER PATIENT**

(57)    A method for predicting responsiveness to therapy for cancer patient is provided, which includes acquiring a pathology slide image of a cancer patient, determining information on a plurality of lymphocytes and information on a plurality of tumor cells included in the pathology slide image, calculating a lymphocyte and tumor cell interaction score based on the information on the plurality of lymphocytes and the information on the plurality of tumor cells, and predicting responsiveness to therapy for the cancer patient by using the interaction score.

FIG. 1

**Description**

**CROSS-REFERENCE TO RELATED APPLICATIONS**

This application claims priority under 35 U.S.C §119 to Korean Patent Application No. 10-2020-0148939 filed in the Korean Intellectual Property Office on November 9, 2020, the entire contents of which are hereby incorporated by reference.

**TECHNICAL FIELD**

The present disclosure relates to a method and a system for predicting responsiveness to therapy for cancer patient. More specifically, the present disclosure provides a method and a system for acquiring histological components from a pathology slide image and predicting responsiveness to therapy for cancer patient based on the acquired histological components.

**BACKGROUND**

Cancer therapy can be divided into a first generation chemotherapy which attacks not only cancer cells but also normal cells, a second generation targeted therapy which selectively attacks only cancer cells, and immunotherapy which activates the immune system such that lymphocytes positioned around cancer tissues selectively attack the tumor cells. In particular, since immunotherapy generally have fewer side effects than existing cancer therapies and have a greater cancer treatment effect, the size of the market is also expected to increase steadily every year, reaching about $48 billion by 2024.

Meanwhile, while cancer therapy costs from several million won to tens of millions of won, it may have side effects in exceptional cases, and may not guarantee therapeutic effect for all cancer patients' tumors. In addition, while the treatment such as chemotherapy, targeted therapy, or the like makes it physically and/or mentally difficult for cancer patients, it is not always effective for the cancer patients. Thus, prior to subjecting a cancer patient to therapy such as immunotherapy, targeted therapy, and the like, it may be important to determine whether or not these therapies will respond appropriately to the cancer patients, that is, whether or not all or at least a part of the cancer patient's tumor can be removed.

**SUMMARY**

The present disclosure has been made to solve the problems described above, and provides a method and a system for predicting responsiveness to therapy for cancer patient.

Provided are a method and a system for predicting responsiveness to therapy for cancer patient by using a pathology slide image of a patient to be subjected to cancer therapy.

Provided are a method and a system for calculating a lymphocyte and tumor cell interaction score (LTS) for predicting responsiveness to therapy for cancer patient associated with a pathology slide image, by using the pathology slide image.

Provided are a method and a system for generating a machine learning model for predicting responsiveness to therapy for cancer patient by using a plurality of pathology slide images acquired from a database of one or more medical systems.

The present disclosure may be implemented in various ways, including a method, a system, an apparatus, or a computer-readable storage medium storing instructions, or a computer program.

According to an embodiment, a method for predicting responsiveness to therapy for cancer patient may include acquiring a pathology slide image of a cancer patient, determining information on a plurality of lymphocytes and information on a plurality of tumor cells included in the pathology slide image, calculating a lymphocyte and tumor cell interaction score (hereinafter, also referred to as "interaction score" or "LTS") based on the information on the plurality of lymphocytes and the information on the plurality of tumor cells, and predicting responsiveness to therapy for the cancer patient by using the interaction score.

According to an embodiment, the determining the information on the plurality of lymphocytes and the information on the plurality of tumor cells may include determining a position of each of the plurality of lymphocytes and a position of each of the plurality of tumor cells, and determining tissue information where each of the plurality of lymphocytes is arranged and tissue information where each of the plurality of tumor cells is arranged, and the calculating the lymphocyte and tumor cell interaction score may include calculating a distance between each of a plurality of lymphocytes belonging to specific tissue information and each of a plurality of tumor cells belonging to the specific tissue information, and calculating the interaction score by using the calculated distance.

According to an embodiment, the determining the information on the plurality of lymphocytes and the information on the plurality of tumor cells may further include determining a characteristic of each of the plurality of lymphocytes and a characteristic of each of the plurality of tumor cells, and the calculating the interaction score by using the calculated distance may include calculating the interaction score by using the determined characteristic of each of the plurality of lymphocytes, the characteristic of each of the plurality of tumor cells, and the calculated distance.

According to an embodiment, the determining the characteristic of each of the plurality of lymphocytes and the characteristic of each of the plurality of tumor cells may include determining a weight according to the characteristic of each of the plurality of lymphocytes and a weight according to the characteristic of each of the plurality of tumor cells, and the calculating the interaction score by using the determined characteristic of each of the plurality of lymphocytes, the characteristic of each of the plurality of tumor cells, and the calculated distance may include calculating the interaction score by using the determined weight according to the characteristic of each of the plurality of lymphocytes, the determined weight according to the characteristic of each of the plurality of tumor cells, and the calculated distance.

According to an embodiment, the calculating the interaction score may include selecting, from among the calculated distance, a distance that is less than or equal to a predetermined threshold associated with interaction, and calculating the interaction score by using the calculated distances.

According to an embodiment, the calculating the interaction score may include determining a higher interaction score as the calculated distance is closer.

According to an embodiment, the predicting the responsiveness to therapy for the cancer patient by using the interaction score may include inputting the interaction score into a machine learning model for responsiveness prediction to output a value indicative of the responsiveness to therapy for the cancer patient.

According to an embodiment, the method may further include acquiring clinical factor on the patient associated with the pathology slide image, in which the inputting the interaction score into the machine learning model for responsiveness prediction may include inputting the acquired clinical factor, the characteristic of at least one of cell, tissue, or structure in the pathology slide image, and the interaction score into the machine learning model for responsiveness prediction to output the value indicative of the responsiveness to therapy for the cancer patient.

According to an embodiment, the inputting the interaction score into the machine learning model for responsiveness prediction may further include normalizing the characteristic of at least one of cell, tissue, or structure in the pathology slide image.

According to an embodiment, a machine learning model for responsiveness prediction may include one of generalized linear machine learning models.

According to an embodiment, an information processing system is provided, which may include a memory storing one or more instructions, and a processor configured to, by executing of the one or more stored instructions, acquire a pathology slide image of a cancer patient, determine information on a plurality of lymphocytes and information on a plurality of tumor cells included in the pathology slide image, calculate a lymphocyte and tumor cell interaction score based on the information on the plurality of lymphocytes and the information on the plurality of tumor cells, and predict responsiveness to therapy for the cancer patient by using the interaction score.

According to an embodiment, the processor may be further configured to determine a position of each of the plurality of lymphocytes and a position of each of the plurality of tumor cells, determine tissue information where each of the plurality of lymphocytes is arranged and tissue information where each of the plurality of tumor cells is arranged, calculate a distance between each of a plurality of lymphocytes belonging to specific tissue information and each of a plurality of tumor cells belonging to the specific tissue information, and calculate the interaction score by using the calculated distance.

According to an embodiment, the processor may be further configured to determine a characteristic of each of the plurality of lymphocytes and a characteristic of each of the plurality of tumor cells, and calculate the interaction score by using the determined characteristic of each of the plurality of lymphocytes, the characteristic of each of the plurality of tumor cells, and the calculated distance.

According to an embodiment, the processor may be further configured to determine a weight according to the characteristic of each of the plurality of lymphocytes and a weight according to the characteristic of each of the plurality of tumor cells, and calculate the interaction score by using the determined weight according to the characteristic of each of the plurality of lymphocytes, the determined weight according to the characteristic of each of the plurality of tumor cells, and the calculated distance.

According to an embodiment, the processor may be further configured to select, from among the calculated distance, a distance that is less than or equal to a predetermined threshold associated with interaction, and calculate the interaction score by using the selected distance.

According to an embodiment, the processor may be further configured to determine a higher interaction score as the calculated distance is closer.

According to an embodiment, the processor may be further configured to input the interaction score into a machine learning model for responsiveness prediction and output a value indicative of the responsiveness to therapy for the cancer patient.

According to an embodiment, the processor may be further configured to acquire clinical factor on the patient associated with the pathology slide image, and input the acquired clinical factor, the characteristic of at least one of cell, tissue, or structure in the pathology slide image, and the interaction score into the machine learning model for responsiveness prediction and output the value indicative of the responsiveness to therapy for the cancer patient.

According to an embodiment, the processor may be further configured to normalize the characteristic of at least one of cell, tissue, or structure in the pathology slide image.

According to an embodiment, a machine learning model for responsiveness prediction may include one of generalized linear machine learning models.

According to some embodiments of the present disclosure, a lymphocyte and tumor cell interaction score is calculated based on a distance between each of a plurality of lymphocytes and each of a plurality of tumor cells present in a specific tissue, and the calculated interaction score may be used to predict responsiveness to therapy for cancer patient, thereby further improving predictive power for the responsiveness to therapy.

According to some embodiments of the present disclosure, in predicting responsiveness to therapy for cancer patient, information on the patient and characteristics of at least one of cell, tissue, or structure in a pathology slide image is used in addition to the lymphocyte and tumor cell interaction score. By using such various information and/or data, a more accurate predictive power for responsiveness to therapy for cancer patient can be provided.

According to some embodiments of the present disclosure, by normalizing information about cell, tissue, and/or structure, and the like within the pathology slide images, inconsistencies and/or heterogeneity of information in pathology slide images that may arise in the course of collecting pathology slide images at different hospitals or systems may be eliminated or reduced. Under this system, responsiveness to therapy for cancer patient can be predicted with reference to pathology slide images collected from various hospitals and/or systems, or information within the images.

**BRIEF DESCRIPTION OF THE DRAWINGS**

Embodiments of the present disclosure will be described with reference to the accompanying drawings described below, where similar reference numerals indicate similar components, but not limited thereto, in which:

FIG. 1 is an exemplary configuration diagram illustrating a system for predicting responsiveness to therapy for cancer patient according to an embodiment;
FIG. 2 is a block diagram illustrating a configuration of a processor that provides a prediction result of responsiveness to therapy for cancer patient according to an embodiment;
FIG. 3 illustrates an example of a method for predicting responsiveness to therapy for cancer patient according to an embodiment;
FIG. 4 illustrates an example of extracting histological components from a pathology slide image according to an embodiment;
FIG. 5 illustrates an example of a method for calculating a lymphocyte and tumor cell interaction score (LTS) according to an embodiment;
FIG. 6 illustrates an example of calculating distances between lymphocytes and tumor cells according to an embodiment;
FIG. 7 illustrates an example of the distances between lymphocytes and tumor cells positioned in a specific tissue according to an embodiment;
FIG. 8 is a diagram illustrating a machine learning model configured to infer or output a prediction result of responsiveness to therapy for cancer patient based on the LTS according to an embodiment;
FIG. 9 is a diagram illustrating a machine learning model configured to infer or output a prediction result of responsiveness to therapy for cancer patient based on LTS, characteristics of at least one of extracted cell, tissue, or structure, and clinical factor, according to another embodiment;
FIG. 10 is a diagram illustrating an example of a data set used in an information processing system according to an embodiment;
FIG. 11 is a structural diagram illustrating an artificial neural network according to an embodiment; and
FIG. 12 is configuration diagram illustrating an exemplary system for predicting responsiveness to therapy for cancer patient according to an embodiment.

**DETAILED DESCRIPTION**

Hereinafter, specific details for the practice of the present disclosure will be described in detail with reference to the accompanying drawings. However, in the following description, detailed descriptions of well-known functions or configurations will be omitted when it may make the subject matter of the present disclosure rather unclear.

In the accompanying drawings, the same or corresponding elements are assigned the same reference numerals. In addition, in the following description of the embodiments, duplicate descriptions of the same or corresponding components may be omitted. However, even if descriptions of elements are omitted, it is not intended that such elements are not included in any embodiment.

Advantages and features of the disclosed embodiments and methods of accomplishing the same will be apparent by referring to embodiments described below in connection with the accompanying drawings. However, the present disclosure is not limited to the embodiments disclosed below, and may be implemented in various different forms, and the present embodiments are merely provided to make the present disclosure complete, and to fully disclose the scope of the invention to those skilled in the art to which the present disclosure pertains.

The terms used herein will be briefly described prior to describing the disclosed embodiments in detail. The terms used herein have been selected as general terms which are widely used at present in consideration of the functions of the present disclosure, and this may be altered according to the intent of an operator skilled in the art, conventional practice, or introduction of new technology. In addition, in specific cases, the term may be arbitrarily selected by the applicant, and the meaning of the term will be described in detail in a corresponding description of the embodiments. Therefore the terms used in the present disclosure should be defined based on the meaning of the terms and the overall content of the present disclosure rather than a simple name of each of the terms.

As used herein, the singular forms "a," "an," and "the" are intended to include the plural forms as well, unless the context clearly indicates the singular forms. Further, the plural forms are intended to include the singular forms as well, unless the context clearly indicates the plural forms. Further, throughout the description, when a portion is stated as "comprising (including)" a component, it intends to mean that the portion may additionally comprise (or include or have) another component, rather than excluding the same, unless specified to the contrary.

Further, the term "module" or "unit" used herein refers to a software or hardware component, and "module" or "unit" performs certain roles. However, the meaning of the "module" or "unit" is not limited to software or hardware. The "module" or "unit" may be configured to be in an addressable storage medium or configured to reproduce one or more processors. Accordingly, as an example, the "module" or "unit" may include components such as software components, object-oriented software components, class components, and task components, and at least one of processes, functions, attributes, procedures, subroutines, program code segments of program code, drivers, firmware, micro-codes, circuits, data, database, data structures, tables, arrays, or variables. Furthermore, functions provided in the components and the "modules" or "units" may be combined into a smaller number of components and "modules" or "units", or further divided into additional components and "modules" or "units."

According to an embodiment, the "module" or "unit" may be implemented as a processor and a memory. The "processor" should be interpreted broadly to encompass a general-purpose processor, a central processing unit (CPU), a microprocessor, a digital signal processor (DSP), a controller, a microcontroller, a state machine, and so forth. Under some circumstances, the "processor" may refer to an application-specific integrated circuit (ASIC), a programmable logic device (PLD), a field-programmable gate array (FPGA), and so on. The "processor" may refer to a combination of processing devices, e.g., a combination of a DSP and a microprocessor, a plurality of microprocessors, one or more microprocessors in conjunction with a DSP core, or any other combination of such configurations. In addition, the "memory" should be interpreted broadly to encompass any electronic component that is capable of storing electronic information. The "memory" may refer to various types of processor-readable media such as random access memory (RAM), read-only memory (ROM), non-volatile random access memory (NVRAM), programmable read-only memory (PROM), erasable programmable read-only memory (EPROM), electrically erasable PROM (EEPROM), flash memory, magnetic or optical data storage, registers, and so on. The memory is said to be in electronic communication with a processor if the processor can read information from and/or write information to the memory. The memory integrated with the processor is in electronic communication with the processor.

In the present disclosure, the "system" may refer to at least one of a server device and a cloud device, but not limited thereto. For example, the system may include one or more server devices. In another example, the system may include one or more cloud devices. In another example, the system may be configured together with both a server device and a cloud device and operated.

In the present disclosure, a "pathology slide image" refers to an image obtained by capturing a pathological slide fixed and stained through a series of chemical treatments in order to observe a tissue removed from a human body with a microscope. In an example, the pathology slide image may refer to a whole slide image including a high-resolution image of the whole slide. Alternatively, the pathology slide image may refer to a part, for example, one or more patches of such whole slide image of high resolution. For example, the pathology slide image may refer to a digital image captured with a microscope, and may include information on cell, tissue, and/or structure in the human body. Further, the pathology slide image may include one or more patches, and the histological components may be applied (e.g., tagged) to the one or more patches through annotation work.

In the present disclosure, the "patch" may refer to a small region within the pathology slide image. For example, the patch may include a region corresponding to a semantic object extracted by performing segmentation on the pathology slide image. As another example, the patch may refer to a combination of pixels associated with the histological components generated by analyzing the pathology slide image.

In the present disclosure, the "histological components" include characteristics or information of cell, tissue, and/or structure in the human body included in the pathology slide image. In this example, the histological components may refer to the histological components of at least one patch included in the pathology slide image, which are inferred through the machine learning model. Alternatively, the histological components may be acquired as a result of the annotator's annotation work.

In the present disclosure, "therapy for cancer patient" may include any therapeutic agent that is taken or administered by the cancer patient and/or any therapeutic treatment operated or applied to the cancer patient. For example, the therapy applied to the cancer patient may include chemotherapy, radiotherapy, immunotherapy, and the like. In addition, the responsiveness to therapy for cancer patient may include pathological complete response, responsiveness to immunotherapy, and the like. In this case, the "pathological complete response (hereinafter, referred to as pCR)" may indicate the absence of invasive cancer in human tissue by chemotherapy or radiotherapy. For example, the pathological complete response may indicate a state in which all or at least a part of the tumor cells present in human tissues are removed as a result of cancer therapy.

In the present disclosure, the "machine learning model" may include any model that is used to infer an answer to a given input. According to an embodiment, the machine learning model may include an artificial neural network model including an input layer, a plurality of hidden layers, and an output layer. In an example, each layer may include one or more nodes. For example, the machine learning model may be trained to infer histological components of pathology slide images and/or at least one patch included in the pathology slide image. In this case, the histological components generated through the annotation work may be used to train the machine learning model. As another example, the machine learning model may be trained to infer responsiveness to therapy for cancer patient based on interaction scores, characteristics of at least one of cell, tissue, or structure in the pathology slide image, and/or clinical factor of the patient. In addition, the machine learning model may include weights associated with a plurality of nodes included in the machine learning model. In an example, the weight may include an any parameter associated with the machine learning model. In the present disclosure, the machine learning model may refer to an artificial neural network model, and the artificial neural network model may refer to the machine learning model.

In the present disclosure, "training" may refer to any process of changing weights included in the machine learning model by using at least one patch, interaction score, histological components, and/or clinical factor. According to an embodiment, the training may refer to a process of changing or updating weights associated with the machine learning model through one or more forward propagations and backward propagations of the machine learning model using at least one patch and the histological components.

In the present disclosure, "annotation" refers to an operation of tagging histological components to a data sample, or to tagged information (that is, annotation) itself. The annotation may be used interchangeably with terms such as tagging, labeling, and so on as used in the art.

In the present disclosure, "each of a plurality of A" may refer to each of all components included in the plurality of A, or may refer to each of some of the components included in a plurality of A. For example, each of a plurality of tumor cells may refer to each of all tumor cells included in the plurality of tumor cells or to each of some tumor cells included in the plurality of tumor cells. Similarly, each of a plurality of lymphocytes may refer to each of all lymphocytes included in the plurality of lymphocytes or may refer to each of some lymphocytes included in the plurality of lymphocytes.

In the present disclosure, "similar" may encompass sameness and similarity. For example, when two pieces of information are similar, it may mean that the two pieces of information are the same as or similar to each other.

In the present disclosure, "instructions" refer to a set of instructions grouped on the basis of function, which are the components of a computer program and executed by a processor.

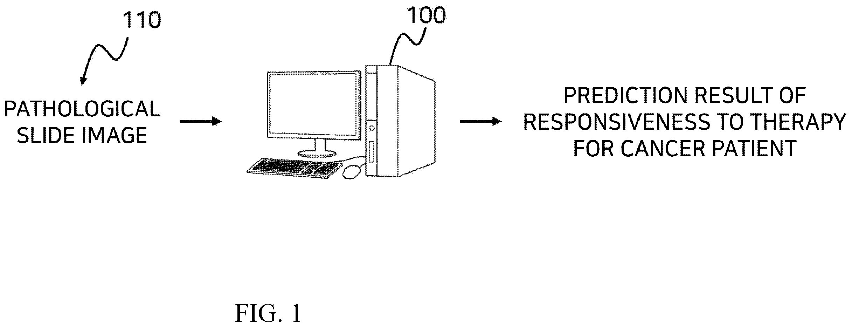

FIG. 1 is an exemplary configuration diagram illustrating a system for providing a prediction result of responsiveness to therapy for cancer patient according to an embodiment. An information processing system 100 may include a system for receiving a pathology slide image 110 and providing a result of predicting responsiveness to therapy for cancer patient. In addition, while the information processing system 100 is illustrated as one computing device in FIG. 1, the present disclosure is not limited thereto, and the information processing system 100 may be configured to process information and/or data in a distributed manner through a plurality of computing devices. In addition, while a storage system 110 is illustrated as a single device in FIG. 1, the present disclosure is not limited thereto, and the system may be configured with a plurality of storage devices or as a system that supports a cloud. In addition, respective components of the system for providing a prediction result of responsiveness to therapy for cancer patient illustrated in FIG. 1 represent functional components that can be divided on the basis of functions, and in an actual physical environment, a plurality of components may be implemented as being incorporated into each other. In addition, although a storage system capable of communicating with the information processing system 100 is not illustrated in FIG. 1, the information processing system 100 may be configured to be connected to or capable of communicating with one or more storage systems.

The information processing system 100 is any computing device used to predict responsiveness to therapy for cancer patient. In an example, the computing device may refer to any type of device equipped with a computing function, and may be a notebook, a desktop, a laptop, a server, a cloud system, and the like, for example, but is not limited thereto.

The information processing system 100 may receive a pathology slide image generated from a human tissue of a patient targeted for prediction of responsiveness to therapy for cancer patient. Such pathology slide image may be received through a communicable storage medium (e.g., hospital system, local/cloud storage system, and the like). The information processing system 100 may predict responsiveness to therapy for cancer patient based on the received pathology slide image. To this end, the information processing system 100 may perform image analysis on the received pathology slide image.

According to another embodiment, the information processing system 100 may utilize the characteristics of cell, tissue, and/or structure in the body of the target patient extracted through such image analysis to provide a prediction result of responsiveness to immunotherapy as the prediction result of responsiveness to therapy for cancer patient. According to another embodiment, the information processing system 100 may utilize the characteristics of cell, tissue, and/or structure in the body of the target patient extracted through such image analysis to provide a prediction result of pathological complete response to cancer therapy as the prediction result of responsiveness to therapy for cancer patient.

The storage system configured to be capable of communicating with the information processing system 100 is an apparatus or a cloud system for storing and managing various data associated with a machine learning model configured to predict responsiveness to therapy for cancer patient from a pathology slide image. For efficient data management, the storage system may store and manage various types of data using a database. In this case, the various data may include any data associated with the machine learning model, and for example, the various data may include the pathology slide image and histological components on the type, position, state, and the like of the cell, tissue, and/or structure included in the pathology slide image. In addition, the various data may include clinical factors such as age, menopause status, clinical T-stage (Clinical_T), BIRADS, number of tumors, tumor size, Node_Enlargement, Biopsy_ER, Biopsy_PR, Biopsy_HER2, pCR_final, and Pathology Type, etc. of the patient.

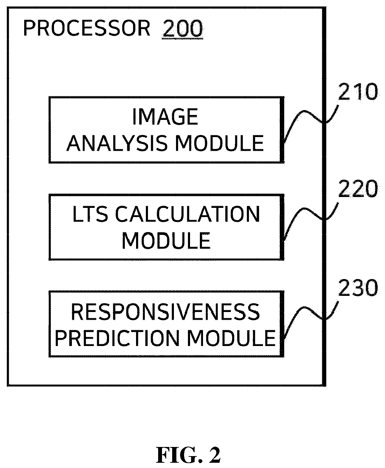

FIG. 2 is a block diagram illustrating a configuration of a processor 200 that provides a result of predicting responsiveness to therapy for cancer patient according to an embodiment. As illustrated, the processor 200 may be configured to include an image analysis module 210, an LTS calculation module 220, and a responsiveness prediction module 230. In addition, although not illustrated in FIG. 2, the processor 200 may transmit and receive various types of information and/or data to and from an external system through a communication interface supporting a wired/wireless Internet service.

The image analysis module 210 may receive an image for analysis from an external system. According to an embodiment, the image analysis module 210 may receive a pathology slide image of a patient targeted for prediction of responsiveness to therapy, from a database of an external system through a communication interface (not illustrated). At this time, the image analysis module 210 may receive a plurality of pathology slide images for a plurality of patients from one external system. Alternatively, the image analysis module 210 may receive a plurality of pathology slide images from each of databases of a plurality of external systems (e.g., systems deployed in different hospitals).

The image analysis module 210 may extract information and/or data included or analyzed in the received image. According to an embodiment, the image analysis module 210 may extract histological components about the type, position, state, and the like of cell, tissue, and/or structure included in the pathology slide image by the machine learning model. According to another embodiment, the image analysis module 210 may provide the pathology slide image to the external system (e.g., an annotator's terminal), and then receive the pathology slide image tagged with the histological components from the external system. The extracted histological components may be provided to the LTS calculation module 220.

The LTS calculation module 220 may calculate the LTS based on the histological components received from the image analysis module 210. According to an embodiment, the LTS calculation module 220 may calculate the LTS based on, from among the extracted histological components, the information on a plurality of lymphocytes and the information on a plurality of tumor cells. In this process, the LTS calculation module 220 may calculate a distance between each of the plurality of lymphocytes belonging to the specific tissue information and each of the plurality of tumor cells belonging to the same tissue information. The calculated distance may be used to calculate the LTS. According to an embodiment, the LTS calculation module 220 may use, from among the extracted histological components, the characteristics of each of a plurality of lymphocytes, the characteristics of each of a plurality of tumor cells, and the calculated distance to calculate an interaction score.

The LTS calculation module 220 may determine a function and a weight used for calculating the LTS based on the normalized histological components. Then, the LTS calculation module 220 may use the determined function and weight to calculate the LTS. According to an embodiment, the LTS calculation module 220 may determine an optimal weight and function by using metrics indicating predictive power, such as AUROC, accuracy, sensitivity, specificity, PPV, NPV, and the like. As used herein, the function may mean any function that can return the histological components received from the image analysis module 210.

The responsiveness prediction module 230 may generate a result of predicting responsiveness to therapy for cancer patient based on the LTS received from the LTS calculation module 220. For example, the responsiveness prediction module 230 may use the received LTS to output a value indicating the prediction result of responsiveness to immunotherapy. As another example, the responsiveness prediction module 230 may use the received LTS to output a value indicating a prediction result of pathological complete response to the cancer therapy.

According to an embodiment, the responsiveness prediction module 230 may use the LTS as an input variable to implement a machine learning model that predicts responsiveness to therapy for cancer patient (e.g., pathological complete response (hereinafter referred to as "pCR") to cancer therapy). According to another embodiment, the responsiveness prediction module 230 may also use at least one of LTS, histological components, and clinical factor as input variables to implement a machine learning model for predicting responsiveness to therapy for cancer patient. For example, such a machine learning model may also implement a machine learning model that receives all of LTS, histological components, and clinical factor as inputs and predicts responsiveness to therapy for cancer patient. In this case, the LTS, the histological components, and the clinical factor may refer to the LTS, the histological components, and the clinical factor calculated and/or inferred from the pathology slide image of the same patient group. The machine learning model described above may use Elastic-Net among the generalized linear machine learning models to implement a model that predicts responsiveness to therapy for cancer patient (e.g., a model that predicts pCR).

The responsiveness prediction module 230 may train the implemented machine learning model and perform validation check of the trained machine learning model. According to an embodiment, the responsiveness prediction module 230 may use a plurality of data sets to perform training and validation check of the machine learning model. For example, the responsiveness prediction module 230 may use the pathology slide image received from the database of the first external system (e.g., "Hospital S") as an input variable to train the machine learning model. Then, the responsiveness prediction module 230 may use the pathology slide image received from the database of the second external system (e.g., "Hospital A") as an input variable to perform validation check of the trained machine learning model. That is, the validity of the machine learning model may be verified through cross validation.

The responsiveness prediction module 230 may perform pre-processing on the received histological components before predicting responsiveness to therapy for cancer patient. According to an embodiment, the LTS calculation module 220 may normalize a characteristic of at least one of cell, tissue, or structure in the pathology slide image. In this case, the LTS calculation module 220 may normalize the histological components of a plurality of pathology slide images received from external systems different from each other. For example, the LTS calculation module 220 may use a trimmed mean of M value (TMM) algorithm and/or Voom normalization to perform normalization on the histological components. This normalized histological components may be used as data for training and/or inference of a machine learning model for predicting responsiveness to therapy for cancer patient. With this configuration, inconsistency and/or heterogeneity or the like of histological components caused by various variables may be eliminated or reduced.

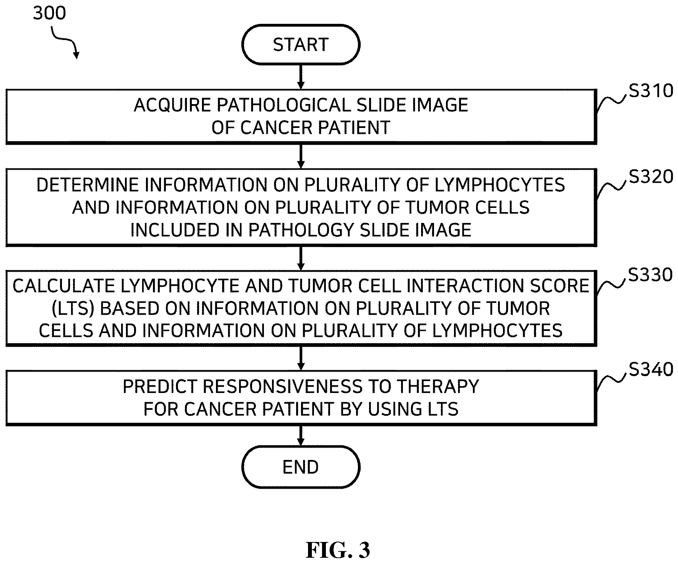

FIG. 3 illustrates an example of a method for predicting responsiveness to therapy for cancer patient according to an embodiment. A method 300 may be performed by at least one processor of an information processing system (e.g., the information processing system 100). The method 300 may be initiated by acquiring a pathology slide image of a cancer patient, at S310.

According to an embodiment, the processor may determine information on a plurality of tumor cells and information on a plurality of lymphocytes included in the pathology slide image, at S320. Then, the processor may calculate an lymphocyte and tumor cell interaction score (LTS) based on the information on the plurality of tumor cells and the information on the plurality of lymphocytes, at S330. Then, the processor may use the LTS to predict responsiveness to therapy for the cancer patient, at S340.

According to an embodiment, the processor may determine a position of each of the plurality of lymphocytes and a position of each of the lymphocytes of the tumor. In addition, the processor may determine tissue information where each of the plurality of lymphocytes is arranged and tissue information where each of the plurality of tumor cells is arranged. Then, the processor may calculate a distance between each of the plurality of lymphocytes belonging to a specific tissue information and each of the plurality of tumor cells belonging to the specific tissue information. By using the distance calculated as described above, an interaction score may be calculated.

According to an embodiment, the processor may be configured to determine a characteristic of each of the plurality of lymphocytes and a characteristic of each of the plurality of tumor cells. The characteristic of each of the plurality of lymphocytes, the characteristic of each of the plurality of tumor cells, and the calculated distance, which are calculated as described above, may be used to calculate the interaction score.

According to an embodiment, the processor may perform an operation of determining a weight according to the characteristic of each of the plurality of lymphocytes and a weight according to the characteristic of each of the plurality of tumor cells. Then, the weight determined according to the characteristic of each of the plurality of lymphocytes, the weight determined according to the characteristic of each of the plurality of tumor cells, and the calculated distance may be used to calculate the interaction score.

According to an embodiment, the processor may select, from among the calculated distance, a distance less than or equal to a predetermined threshold associated with the interaction. The processor may then use the selected distance to calculate the interaction score. In this case, the closer the calculated distance is, the higher the interaction score may be determined.

According to an embodiment, the processor may input the interaction score into the machine learning model for predicting responsiveness to therapy for cancer patient, and output a value indicative of the responsiveness to therapy for the cancer patient associated with the pathology slide image. To this end, the processor may normalize a characteristic of at least one of cell, tissue, or structure in the pathology slide image. According to another embodiment, the processor may acquire clinical factor on the patient associated with the pathology slide image, and input the acquired clinical factor, the characteristic of at least one of cell, tissue, or structure in the pathology slide image, and the interaction score into a machine learning model for responsiveness prediction to therapy for cancer patient to output the value indicative of the responsiveness to therapy for the cancer patient associated with the pathology slide image. In this case, the machine learning model for responsiveness prediction may include one of generalized linear machine learning models.

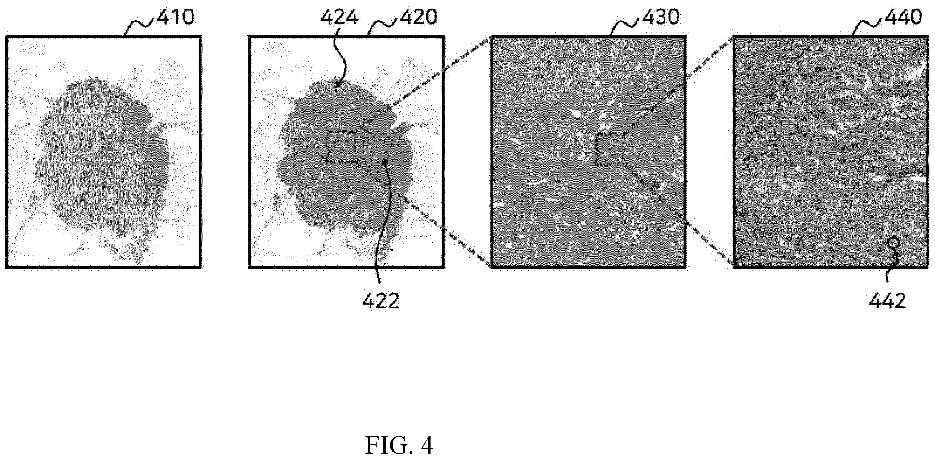

FIG. 4 illustrates an example of extracting histological components from a pathology slide image according to an embodiment. As illustrated, a pathology slide image 410 may refer to a digital image generated by imaging a pathological slide of at least a part of the tissue obtained from the human body, which is stained and/or fixed through a series of chemical treatments, with at least one of a slide scanner, a microscope, and a camera. In FIG. 4, the pathology slide image 410 is illustrated as being stained by hematoxylin and eosin (H&E) staining technique, but is not limited thereto, and may include an image generated by imaging a pathological slide stained with a different known staining technique with at least one of slide scanner, microscope, and camera. In this case, the slide scanner may include a digital pathology slide scanner.

A pathology slide image 420 may include histological components for at least one patch 430 included in the image. According to an embodiment, an image analysis module (e.g., the image analysis module 210) may generate histological components on at least one patch included in the pathology slide image by analyzing (e.g., inferring) the pathology slide image 410 with the machine learning model. In another embodiment, the histological components may be generated by a human (e.g., an annotator) performing annotation work on the at least one patch.

For the purpose of pathological determination, the histological components may include information for distinguishing a target included in the pathology slide image 420 and/or the first patch 430. The pathology slide image 420 may include histological components for a specific region. According to an embodiment, histological components of cancer stroma may be extracted from a first region 422 in the pathology slide image 420, and histological components of cancer epithelium may be extracted from a second region 424. For example, as illustrated, the first region 422 corresponding to the cancer stroma region is colored purple, and the second region 424 corresponding to the cancer epithelium region is colored sky blue, but embodiments are not limited thereto, and the histological components may be expressed in various other visual representations such as regions, figures, different colors, or texts. In addition, all or at least part of the colored region may correspond to one or more patches.

In an embodiment, information on lymphocytes 442 (e.g., the number of lymphocytes, position where the lymphocytes are arranged, and the like) may be extracted as histological components into the patch in the pathology slide image. As illustrated, the lymphocytes 442 may include one or more lymphocytes arranged in cancer epithelial tissue. The distance between each of the lymphocytes arranged in the cancer epithelial tissue and each of the tumor cells in the cancer epithelial tissue may be calculated, and the lymphocyte and tumor cell interaction score, that is, LTS, may be calculated based on the calculated distance.

According to an embodiment, when the pathology slide image 420 is enlarged at a magnification similar to that of a second patch 440, the lymphocytes 442 included in the pathological slide (e.g., helper T cells, killer T cells, natural killer T cells, memory T cells, suppressor T cells and B cells, and the like) may be identified, but embodiments are not limited thereto, and the image analysis module may provide a visual representation on a low magnification image in a region corresponding to the specific histological components. Further, in addition to the lymphocytes 442, the histological components may include various cells included in the target in the pathological slide, such as neutrophils, eosinophils, basophils, monocytes, red blood cells, platelets, or the like.

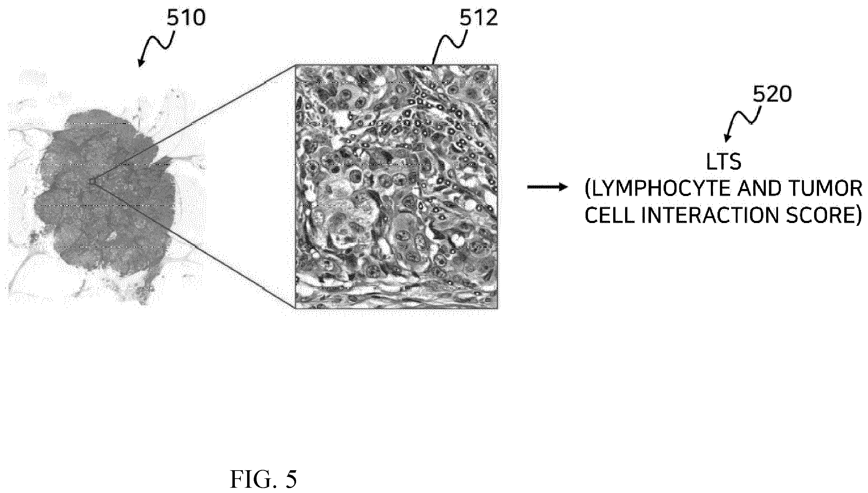

FIG. 5 illustrates an example of a method for calculating an LTS according to an embodiment. The histological components used for LTS calculation may be extracted from a patch 512 belonging to a pathology slide image 510. For example, the patch 512 may include information on type of cells (e.g., erated & necrotic tumor cell, others, endothelial cell and pericyte, mitosis, macrophage, lymphoplasma cell, fibroblast, nuclear grade 1, nuclear grade 2, nuclear grade 3), coordinate values, severity, and the like. In addition, the extracted histological components may include information on tissue in the patch, such as cancer epithelium, cancer stroma, normal epithelium, normal stroma, and necrosis, fat, background, and the like, for example. In addition, the histological components on the patch may include information on structure, such as tubule formation count, tubule formation area, DCIS count, DCIS area, nerve count, nerve area, blood vessel count, blood vessel area, and the like, for example. In addition, the histological components extracted from the patch are not limited to the examples described above, and may include any histological components that can be quantified in the patch, such as cell instability, cell cycle, biological function, and the like.

According to an embodiment, a processor (e.g., the processor 200) may calculate an LTS 520 based on the histological components included in the pathology slide image 510. In this case, the LTS 520 may be calculated with an LTS calculation function using the position information of a plurality of lymphocytes and the position information of a plurality of tumor cells included in the pathology slide image 510 as input variables. More specifically, the LTS may be expressed as the number of distances having a value less than or equal to a predetermined threshold, among the distance between each of the plurality of lymphocytes and each of the tumor cells belonging to the specific tissue. For example, the LTS may be calculated by using, as an input variable of the LTS calculation function, a value obtained by counting the distances having a value less than or equal to 1000px (or 200 μm) with respect to a combination of all lymphocytes and tumor cells belonging to cancer stroma.

According to another embodiment, the LTS 520 may be calculated by using the histological components of lymphocytes and tumor cells and weights inferred based on the histological components. For example, the LTS 520 may be calculated by using Equation 1 below.

<Equation 1>

$$LTS = \sum_{i=1}^{i=n_t} \sum_{j=1}^{j=n_t} \frac{\beta_{ij}}{\alpha * F(L_i, T_j)} \Big/ \sum_{i=1}^{i=n_t} \beta$$

where, the function F in Equation 1 may refer to any function that can be inferred based on histological characteristics and phase information of lymphocytes and tumor cells. For example, the function F may refer to a distance function that uses the position $L_i$ of lymphocytes and the position $T_j$ of tumor cells as input variables. As another example, the function F may be expressed as an (n)th-order equation for the calculated distance. As still another example, the function F may refer to a formula having an output value that is changed according to characteristics of lymphocytes and/or characteristics of tumor cells. In addition, the weight $\alpha$ may refer to a weight for each distance between lymphocyte and tumor cell. In addition, the weight $\beta_{ij}$ may mean a weight for characteristics associated with cellular phenomena of lymphocytes and tumor cells (e.g., Grade 1/2/3 of tumor cells, mitosis, and the like). In addition, the weight $\beta$ may refer to a weight for characteristic of each of lymphocytes and/or tumor cells in addition to the above characteristics. In this case, the weight $\beta$ may have different weights according to certain state information, and for example, a weight most suitable for calculating the LTS may be determined through experimentation and/or learning.

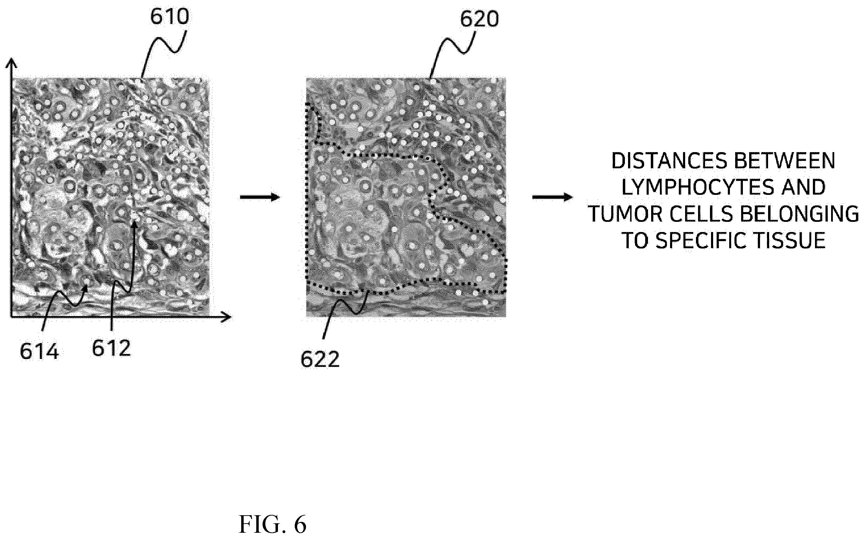

FIG. 6 illustrates an example of calculating distances between lymphocytes and tumor cells according to an embodiment. According to an embodiment, the information processing system (e.g., the information processing system 100) may determine a position of each cell positioned in a pathology slide image 610. For example, the information processing system may use histological components on the types of cells extracted or tagged in the pathology slide image 610 to determine the position information of each of lymphocytes 612 and tumor cells 614. In this case, the position information of each of the lymphocytes 612 and the tumor cells 614 may be expressed as pixel positions or coordinate values (values representing coordinates from a specific reference pixel) on the pathology slide image.

According to an embodiment, the information processing system may determine, from a pathology slide image 620, information on a region in which cells are arranged. For example, the information processing system may use the extracted information on the tissue (e.g., cancer epithelium, cancer stroma, normal epithelium, normal stroma, necrosis, fat, background, and the like) from the pathology slide image 610 to extract information on the tissue where the extracted lymphocytes 612 and the tumor cells 614 are positioned. For example, the tissue information (e.g., cancer stroma) of tissue region 622 in which the lymphocytes 612 and the tumor cells 614 are positioned may be determined.

According to an embodiment, the information processing system may calculate a distance between each of the lymphocytes and each of the tumor cells based on the position of the tumor cell 614 and the position of the lymphocyte 612 in the specific tissue region 622. For example, the information processing system may calculate a distance between each of the lymphocytes and each of the tumor cells positioned in the cancer epithelium. As another example, the information processing system may calculate a distance between each of the lymphocytes and each of the tumor cells positioned in the cancer stroma.

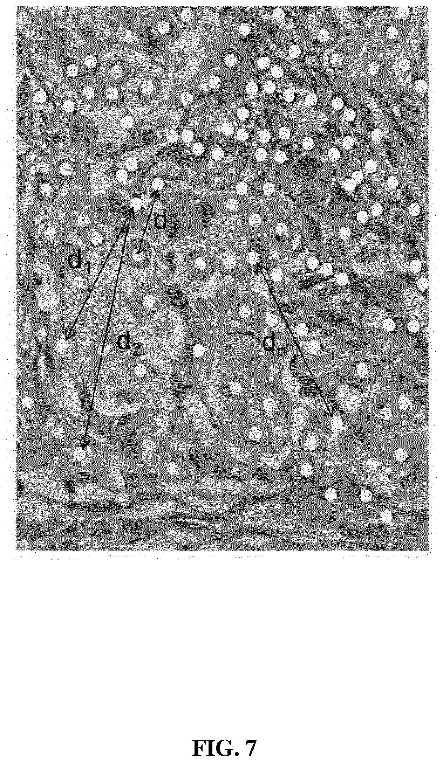

FIG. 7 illustrates an example of the distances between lymphocytes and tumor cells positioned in a specific tissue according to an embodiment. The information processing system (e.g., the information processing system 100) may calculate a distance between each of the lymphocytes and each of the tumor cells belonging to specific tissue information based on the histological components included in the pathology slide image. The specific tissue information herein may refer to cancer stroma or cancer epithelium, but is not limited thereto, and may also refer to tissue information of any one of normal epithelium, normal stroma, necrosis, fat, and background.

According to an embodiment, the information processing system may calculate a distance (di, d2, d3, ..., $d_n$) between each of all lymphocytes and each of all tumor cells belonging to specific tissue information. According to another embodiment, the information processing system may calculate a distance ($d_i$, $d_2$, $d_3$, ..., $d_{n-k}$, k is natural number) between each of at least a part of lymphocytes and each of at least a part of tumor cells belonging to specific tissue information. Here, n may mean the number of combinations of lymphocytes and tumor cells belonging to specific tissue information, which is used to calculate the distances.

Among the calculated distance between each of the lymphocytes and each of the tumor cells for calculating the LTS, the information processing system may use only a distance having a value less than or equal to a predetermined threshold. For example, when the predetermined threshold has a value greater than $d_1$ and less than d2, the information processing system may use the distance $d_1$ between any first lymphocyte (sky blue) and the first tumor cell (yellow) belonging to the cancer stroma (blue region) for calculating the LTS. In addition, the information processing system may not use the distance d2 between the first lymphocyte (sky blue) and the second tumor cell (yellow) for calculating the LTS. With this configuration, only the data related to the distances between lymphocytes and tumor cells that affect the prediction of responsiveness to therapy for cancer patient may be selectively used as input variables for predicting responsiveness to therapy for cancer patient.

The information processing system may use the weight determined for each of the calculated distances to calculate the LTS. For example, the LTS may be expressed by Equation 1 described with reference to FIG. 5. In this case, the function F in Equation 1 may refer to a function that uses the distances between the lymphocytes and the tumor cells calculated based on the positions of the lymphocytes and the tumor cells. In this case, the first weight $\alpha_1$ of the distance (F = di) between the first lymphocyte (sky blue) and the first tumor cell (yellow) may have a different value from the second weight $\alpha_2$ of the distance (F = $d_2$) between the first lymphocyte (sky blue) and the second tumor cell (yellow). For example, the first weight $\alpha_1$ may have a greater value than the second weight $\alpha_2$. According to such a configuration, it is possible to calculate the LTS reflecting the correlation between the distances between lymphocytes and tumor cells and responsiveness to therapy for cancer patient.

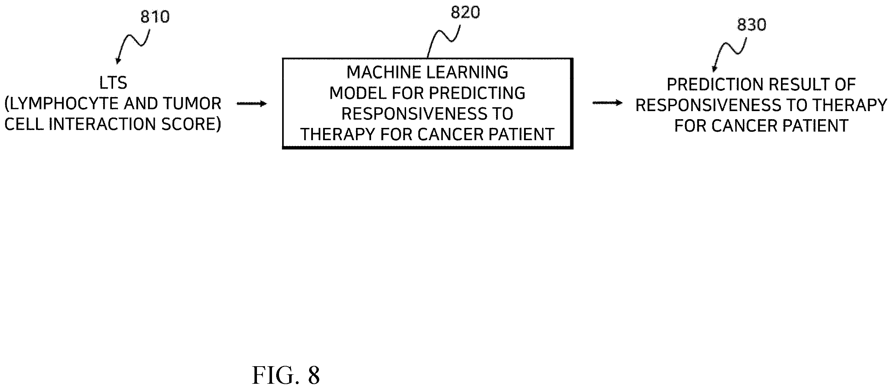

FIG. 8 is a diagram illustrating a machine learning model 820 configured to infer or output a prediction result 830 of responsiveness to therapy for cancer patient based on an LTS 810 according to an embodiment. According to an embodiment, the machine learning model 820 may be generated and updated through a responsiveness prediction module (e.g., the responsiveness prediction module 230). In addition, the responsiveness prediction module may access the machine learning model 820 to infer the prediction result 830 of responsiveness. For example, the responsiveness prediction module may include a model that uses Elastic-Net among the generalized linear models to output prediction result of responsiveness to therapy for cancer patient. In this case, the LTS 810 calculated by the LTS calculation module (e.g., the LTS calculation module 220) may be utilized as training data of the machine learning model 820. The machine learning model 820 generated as described above may be stored in a storage medium (e.g., memory) accessible by the processor.

The machine learning model 820 may use the LTS 810 calculated by the LTS calculation module as input data to infer and/or output the prediction result 830 for responsiveness to therapy for cancer patient. For example, the machine learning model 820 may infer and/or output a prediction result of responsiveness to immunotherapy as the prediction result 830 for responsiveness to therapy for cancer patient. As another example, the machine learning model 820 may infer and/or output a prediction result of pathological complete response to cancer therapy as the prediction result 830 for responsiveness to therapy for cancer patient.

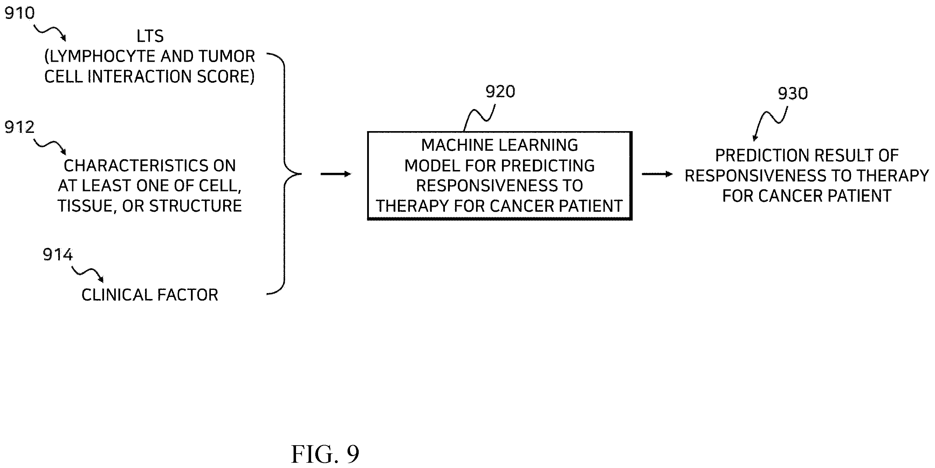

FIG. 9 is a diagram illustrating a machine learning model 920 configured to infer or output a prediction result 930 of responsiveness to therapy for cancer patient based on LTS 910, characteristics 912 on at least one of cell, tissue, or structure, and clinical factor 914, according to another embodiment. According to an embodiment, for the machine learning model, a model for predicting responsiveness to therapy for cancer patient may be constructed, which uses Elastic-Net among the generalized linear models to output prediction result of responsiveness to therapy for cancer patient. In this case, the LTS 910 calculated by the LTS calculation module (e.g., the LTS calculation module 220), the characteristics 912 of at least one of cell, tissue, or structure extracted from the pathological slide through the image analysis module (e.g., the image analysis module 210) (e.g., characteristics of lymphocyte, background, tumor cell, and the like), and the clinical factor 914 of the patient associated with the pathological slide received from the accessible external system may be utilized as the training data. Then, the generated machine learning model 920 may be stored in a storage medium (e.g., memory) accessible by the processor.

The machine learning model 920 may use, as input data, at least one of the LTS 910 calculated by the LTS calculation module, the characteristics 912 of at least one of cell, tissue, or structure extracted from the pathological slide through the image analysis module, and the clinical factor 914 of the patient associated with the pathology slide received from an accessible external system, to infer and/or output the prediction result 930 of responsiveness to therapy for cancer patient. For example, the machine learning model 920 may infer and/or output a prediction result of responsiveness to immunotherapy as the prediction result 930 for responsiveness to therapy for cancer patient. As another example, the machine learning model 920 may infer and/or output a prediction result of pathological complete response to cancer therapy as the prediction result 930 for responsiveness to therapy for cancer patient.

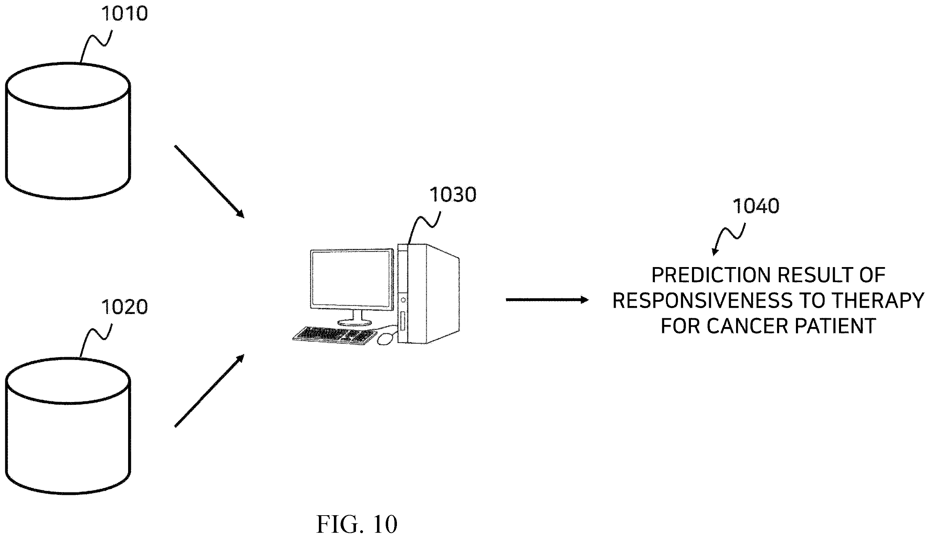

FIG. 10 is a diagram illustrating an example of a data set used in an information processing system 1030 according to an embodiment. The information processing system 1030 may herein correspond to the information processing system 100 of FIG. 2. As illustrated, the data set used for the machine learning model for predicting responsiveness to therapy for cancer patient may be classified into a training data set 1010 and a testing data set 1020. According to an embodiment, the information processing system 1030 may use the training data set 1010 received from the first external system (e.g., system of Hospital S) to train the machine learning model.

According to an embodiment, the information processing system 1030 may input the testing data set 1020 to the trained machine learning model to infer a prediction result 1040 for responsiveness to therapy for cancer patient for the testing data set 1020. According to this configuration, the information processing system 1030 may generate an optimal machine learning model of which validity is verified for a plurality of data sets through cross validation. Additionally, the information processing system 1030 may evaluate the performance of the implemented machine learning model through numerical values related to the performance of the machine learning model, such as prediction accuracy (Accuracy (ACC), area under curve (AUC), sensitivity, specificity, and the like.

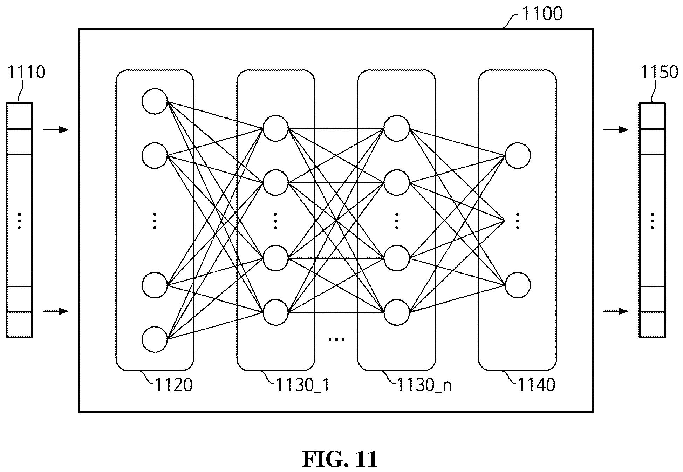

FIG. 11 is a structural diagram illustrating an artificial neural network 1110 according to an embodiment. In machine learning technology and cognitive science, an artificial neural network 1100 refers to a statistical training algorithm implemented based on a structure of a biological neural network, or to a structure that executes such algorithm. That is, the artificial neural network 1100 represents a machine learning model that acquires a problem solving ability by repeatedly adjusting the weights of synapses by the nodes that are artificial neurons forming the network through synaptic combinations as in the biological neural networks, thus training to reduce errors between a correct output corresponding to a specific input and an inferred output.

In general, the artificial neural network is implemented as a multilayer perceptron (MLP) formed of multiple nodes and connections between them. The artificial neural network 1100 according to the present embodiment may be implemented using one of various artificial neural network structures including the MLP. As shown in FIG. 11, the artificial neural network 1100 includes an input layer 1120 receiving an input signal or data 1110 from the outside, an output layer 1140 outputting an output signal or data 1150 corresponding to the input data, and (n) number of hidden layers 1130_1 to 1130_n positioned between the input layer 1120 and the output layer 1140 to receive a signal from the input layer 1120, extract the features, and transmit the features to the output layer 1140. Here, the output layer 1140 receives signals from the hidden layers 1130_1 to 1130_n and outputs them to the outside. In general, the training method of the artificial neural network 1100 includes a supervised learning that trains for optimization for solving a problem with inputs of teacher signals (correct answer), and an unsupervised learning that does not require a teacher signal. The method and system for predicting responsiveness to therapy for cancer patient according to the present disclosure can train the artificial neural network 1100 for extracting prediction result of responsiveness to therapy for cancer patient using supervised learning, unsupervised learning, and semi-supervised learning. The artificial neural network 1100 trained as described above may extract the prediction result of responsiveness to therapy for cancer patient related to the corresponding pathology slide image.

According to an embodiment, as illustrated in FIG. 11, an input variable of the artificial neural network 1100 capable of extracting histological components may be a pathology slide image vector 1110 formed of a pathology slide image as one vector data element. According to an embodiment, the output variable may be formed of a result vector 1150 indicating characteristics of at least one of cell, tissue, and structure in the pathology slide image.

According to another embodiment, the artificial neural network 1100 may be trained to extract responsiveness to therapy for cancer patient according to the input variable. For example, this input variable may be a vector 1110 formed of one vector data element representing the LTS. As another example, this input variable may be the vector 1110 formed of one vector data element representing the LTS, the characteristics of at least one of extracted cell, tissue, or structure, and clinical factor. In addition, the output variable of the artificial neural network 1100 may be formed of the result vector 1150 representing a result of predicting responsiveness to therapy for cancer patient. For example, the result vector 1150 may include a vector indicative of responsiveness to immunotherapy. As another example, the result vector 1150 may include a vector indicating whether or not pathological complete response to the cancer therapy is indicated.

As described above, the input layer 1120 and the output layer 1140 of the artificial neural network 1100 are respectively matched with a plurality of output variables corresponding to a plurality of input variables, so as to adjust the synaptic values between nodes included in the input layer 1120, the hidden layers 1130_1 to 1130_n, and the output layer 1140, thereby enabling training to infer the correct output corresponding to a specific input. Through this training process, the features hidden in the input variables of the artificial neural network 1100 may be confirmed, and the synaptic values (or weights) between the nodes of the artificial neural network 1100 may be adjusted so as to reduce the errors between the output variable calculated based on the input variable and the target output.

By using the artificial neural network 1100 trained as described above, it is possible to extract a prediction result of responsiveness to therapy for cancer patient from the pathology slide image of the cancer patient. For example, by using the artificial neural network 1100, it is possible to extract the prediction result of responsiveness to immunotherapy from the pathology slide image of the cancer patient. As another example, by using the artificial neural network 1100, it

EP 3 998 586 A1

is possible to extract the prediction result of pathological complete response to cancer therapy from the pathology slide image of the cancer patient.

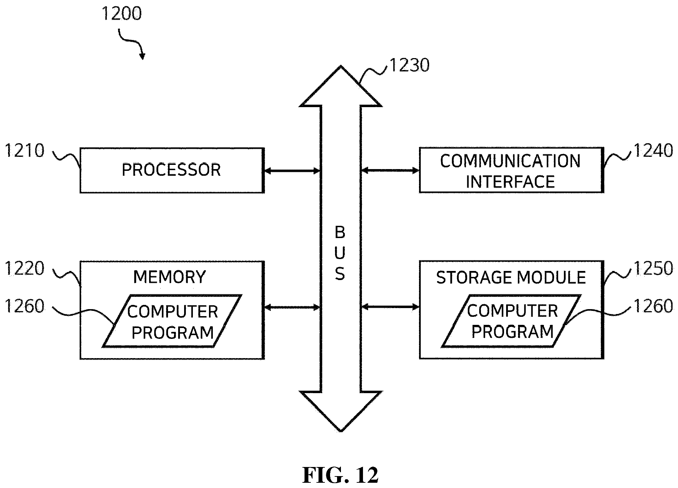

FIG. 12 is configuration diagram illustrating an exemplary system for predicting responsiveness to therapy for cancer patient according to an embodiment. As illustrated, the information processing system 100 may include one or more processors 1210, a bus 1230, a communication interface 1240, a memory 1220 for loading a computer program 1260 to be executed by the processors 1210, and a storage module 1250 for storing the computer program 1260. However, only the components related to the embodiment are illustrated in FIG. 12. Accordingly, those of ordinary skill in the art to which the present disclosure pertains will be able to recognize that other general-purpose components may be further included in addition to the components shown in FIG. 12.

The processors 1210 control the overall operation of components of the information processing system (e.g., the information processing system 100). The processors 1210 may be configured to include a central processing unit (CPU), a microprocessor unit (MPU), a micro controller unit (MCU), a graphic processing unit (GPU), or any type of processor well known in the technical field of the present disclosure. In addition, the processors 1210 may perform an arithmetic operation on at least one application or program for executing the method according to the embodiments of the present disclosure. The information processing system may include one or more processors.

The memory 1220 may store various types of data, commands, and/or information. The memory 1220 may load one or more computer programs 1260 from the storage module 1250 in order to execute the method/operation according to various embodiments of the present disclosure. The memory 1220 may be implemented as a volatile memory such as RAM, although the technical scope of the present disclosure is not limited thereto.

The bus 1230 may provide a communication function between components of the information processing system. The bus 1230 may be implemented as various types of buses such as an address bus, a data bus, a control bus, or the like.

The communication interface 1240 may support wired/wireless Internet communication of the information processing system. In addition, the communication interface 1240 may support various other communication methods in addition to the Internet communication. To this end, the communication interface 1240 may be configured to include a communication module well known in the technical field of the present disclosure.

The storage module 1250 may non-temporarily store one or more computer programs 1260. The storage module 1250 may be configured to include a nonvolatile memory such as a read only memory (ROM), an erasable programmable ROM (EPROM), an electrically erasable programmable ROM (EEPROM), a flash memory, and the like, a hard disk, a detachable disk, or any type of computer-readable recording medium well known in the art to which the present disclosure pertains.

The computer program 1260 may include one or more instructions that, when loaded into the memory 1220, cause the processors 1210 to perform an operation/method in accordance with various embodiments of the present disclosure. That is, the processors 1210 may perform operations/methods according to various embodiments of the present disclosure by executing one or more instructions.

For example, the computer program 1260 may include one or more instructions for causing operations to be performed, the operations including: an operation of determining information on a plurality of lymphocytes and information on a plurality of tumor cells included in the pathology slide image; an operation of calculating LTS based on the information on the plurality of lymphocytes and the information on the plurality of tumor cells; and an operation of predicting responsiveness to therapy for cancer patient by using the LTS. In this case, a system for predicting responsiveness to therapy for cancer patient according to some embodiments of the present disclosure may be implemented through the information processing system 100.

The above description of the present disclosure is provided to enable those skilled in the art to make or use the present disclosure. Various modifications of the present disclosure will be readily apparent to those skilled in the art, and the general principles defined herein may be applied to various modifications without departing from the spirit or scope of the present disclosure. Thus, the present disclosure is not intended to be limited to the examples described herein but is intended to be accorded the broadest scope consistent with the principles and novel features disclosed herein.

Although example implementations may refer to utilizing aspects of the presently disclosed subject matter in the context of one or more standalone computer systems, the subject matter is not so limited, and they may be implemented in conjunction with any computing environment, such as a network or distributed computing environment. Furthermore, aspects of the presently disclosed subject matter may be implemented in or across a plurality of processing chips or devices, and storage may be similarly influenced across a plurality of devices. Such devices may include PCs, network servers, and handheld devices.

Although the present disclosure has been described in connection with some embodiments herein, it should be understood that various modifications and changes can be made without departing from the scope of the present disclosure, which can be understood by those skilled in the art to which the present disclosure pertains. In addition, such modifications and changes should be considered within the scope of the claims appended herein.

**Claims**

1. A method for predicting responsiveness to therapy for cancer patient, comprising:

   acquiring a pathology slide image of a cancer patient;
   determining information on a plurality of lymphocytes and information on a plurality of tumor cells included in the pathology slide image;
   calculating a lymphocyte and tumor cell interaction score based on the information on the plurality of lymphocytes and the information on the plurality of tumor cells; and
   predicting responsiveness to therapy for the cancer patient by using the interaction score.

2. The method according to claim 1, wherein the determining the information on the plurality of lymphocytes and the information on the plurality of tumor cells includes:

   determining a position of each of the plurality of lymphocytes and a position of each of the plurality of tumor cells; and
   determining tissue information where each of the plurality of lymphocytes is arranged and tissue information where each of the plurality of tumor cells is arranged, and
   the calculating the lymphocyte and tumor cell interaction score includes:

   calculating a distance between each of a plurality of lymphocytes belonging to specific tissue information and each of a plurality of tumor cells belonging to the specific tissue information; and
   calculating the interaction score by using the calculated distance.

3. The method according to claim 2, wherein the determining the information on the plurality of lymphocytes and the information on the plurality of tumor cells further includes:

   determining a characteristic of each of the plurality of lymphocytes and a characteristic of each of the plurality of tumor cells, and
   the calculating the interaction score by using the calculated distance includes:
   calculating the interaction score by using the determined characteristic of each of the plurality of lymphocytes, the characteristic of each of the plurality of tumor cells, and the calculated distance.

4. The method according to claim 3, wherein the determining the characteristic of each of the plurality of lymphocytes and the characteristic of each of the plurality of tumor cells includes:

   determining a weight according to the characteristic of each of the plurality of lymphocytes and a weight according to the characteristic of each of the plurality of tumor cells, and
   the calculating the interaction score by using the determined characteristic of each of the plurality of lymphocytes, the characteristic of each of the plurality of tumor cells, and the calculated distance includes:
   calculating the interaction score by using the determined weight according to the characteristic of each of the plurality of lymphocytes, the determined weight according to the characteristic of each of the plurality of tumor cells, and the calculated distance.

5. The method according to claim 1, wherein the calculating the interaction score includes:

   selecting, from among the calculated distance, a distance that is less than or equal to a predetermined threshold associated with interaction; and
   calculating the interaction score by using the selected distance.

6. The method according to claim 1, wherein the calculating the interaction score includes:

   determining a higher interaction score as the calculated distance is closer.

7. The method according to claim 1, wherein the predicting the responsiveness to therapy for the cancer patient by using the interaction score includes:
   inputting the interaction score into a machine learning model for responsiveness prediction to output a value indicative of the responsiveness to therapy for the cancer patient.

8. The method according to claim 7, further comprising acquiring clinical factor on the patient associated with the pathology slide image, wherein
the inputting the interaction score into the machine learning model for responsiveness prediction includes:
inputting the acquired clinical factor, the characteristic of at least one of cell, tissue, or structure in the pathology slide image, and the interaction score into the machine learning model for responsiveness prediction to output the value indicative of the responsiveness to therapy for the cancer patient.

9. The method according to claim 8, wherein the inputting the interaction score into the machine learning model for responsiveness prediction further includes:
Normalizing the characteristic of at least one of cell, tissue, or structure in the pathology slide image.

10. The method according to claim 7, wherein the machine learning model for responsiveness prediction includes one of generalized linear machine learning models.

11. An information processing system comprising:

a memory storing one or more instructions; and
a processor configured to, by executing the one or more stored instructions:
acquire a pathology slide image of a cancer patient; determine information on a plurality of lymphocytes and information on a plurality of tumor cells included in the pathology slide image; calculate a lymphocyte and tumor cell interaction score based on the information on the plurality of lymphocytes and the information on the plurality of tumor cells; and predict responsiveness to therapy for the cancer patient by using the interaction score.

12. The information processing system according to claim 11, wherein the processor is further configured to:
determine a position of each of the plurality of lymphocytes and a position of each of the plurality of tumor cells; determine tissue information where each of the plurality of lymphocytes is arranged and tissue information where each of the plurality of tumor cells is arranged; calculate a distance between each of a plurality of lymphocytes belonging to specific tissue information and each of a plurality of tumor cells belonging to the specific tissue information; and calculate the interaction score by using the calculated distance.

13. The information processing system according to claim 12, wherein the processor is further configured to:
determine a characteristic of each of the plurality of lymphocytes and a characteristic of each of the plurality of tumor cells; and calculate the interaction score by using the determined characteristic of each of the plurality of lymphocytes, the characteristic of each of the plurality of tumor cells, and the calculated distance.

14. The information processing system according to claim 13, wherein the processor is further configured to:
determine a weight according to the characteristic of each of the plurality of lymphocytes and a weight according to the characteristic of each of the plurality of tumor cells; and calculate the interaction score by using the determined weight according to the characteristic of each of the plurality of lymphocytes, the determined weight according to the characteristic of each of the plurality of tumor cells, and the calculated distance.

15. The information processing system according to claim 11, wherein the processor is further configured to:
select, from among the calculated distance, a distance that is less than or equal to a predetermined threshold associated with interaction; and calculate the interaction score by using the selected distance.

16. The information processing system according to claim 11, wherein the processor is further configured to:
determine a higher interaction score as the calculated distance is closer.

17. The information processing system according to claim 11, wherein the processor is further configured to:
input the interaction score into a machine learning model for responsiveness prediction and output a value indicative of the responsiveness to therapy for the cancer patient.

18. The information processing system according to claim 17, wherein the processor is further configured to:
Acquire clinical factor on the patient associated with the pathology slide image; and input the acquired clinical factor, the characteristic of at least one of cell, tissue, or structure in the pathology slide image, and the interaction score into the machine learning model for responsiveness prediction to output the value indicative of the responsiveness to therapy for the cancer patient.

**19.** The information processing system according to claim 18, wherein the processor is further configured to:
normalize the characteristic of at least one of cell, tissue, or structure in the pathology slide image.

**20.** The information processing system according to claim 17, wherein a machine learning model for responsiveness prediction includes one of generalized linear machine learning models.

FIG. 1

EP 3 998 586 A1

PROCESSOR 200

IMAGE
ANALYSIS MODULE  210

LTS CALCULATION
MODULE  220

RESPONSIVENESS
PREDICTION MODULE  230

**FIG. 2**

FIG. 3

FIG. 4

FIG. 5

DISTANCES BETWEEN
LYMPHOCYTES AND
TUMOR CELLS BELONGING
TO SPECIFIC TISSUE

FIG. 6

**FIG. 7**

EP 3 998 586 A1

810

LTS
(LYMPHOCYTE AND TUMOR
CELL INTERACTION SCORE)

→

820

MACHINE LEARNING
MODEL FOR PREDICTING
RESPONSIVENESS TO
THERAPY FOR CANCER PATIENT

→

830

PREDICTION RESULT OF
RESPONSIVENESS TO THERAPY
FOR CANCER PATIENT

FIG. 8

910 LTS
(LYMPHOCYTE AND TUMOR
CELL INTERACTION SCORE)

912 CHARACTERISTICS ON
AT LEAST ONE OF CELL,
TISSUE, OR STRUCTURE

914 CLINICAL FACTOR

920 MACHINE LEARNING
MODEL FOR PREDICTING
RESPONSIVENESS TO
THERAPY FOR CANCER PATIENT

930 PREDICTION RESULT OF
RESPONSIVENESS TO THERAPY
FOR CANCER PATIENT

FIG. 9

EP 3 998 586 A1

1010

1020

1030

1040

PREDICTION RESULT OF
RESPONSIVENESS TO THERAPY
FOR CANCER PATIENT

FIG. 10

**FIG. 11**

FIG. 12

Europäisches
Patentamt

European
Patent Office

Office européen
des brevets

# EUROPEAN SEARCH REPORT

**Application Number**

EP 21 20 7007

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/258223 A1 (YIP STEPHEN [US] ET AL) 13 August 2020 (2020-08-13) * abstract * * paragraph [0018] - paragraph [0022] * * paragraph [0046] * * paragraph [0086] * * paragraph [0090] - paragraph [0093] * * paragraph [0098] * * paragraph [0187] * * paragraph [0254] * ----- | 1-20 | INV. G06V20/69 G06V10/82 G06V10/74 G06K9/62 |
| A | WO 2020/083970 A1 (HOFFMANN LA ROCHE [US]; HOFFMANN LA ROCHE [CH]) 30 April 2020 (2020-04-30) * abstract * * page 40, line 16 - page 41, line 14 * ----- | 2-6, 12-16 | |
| | | | TECHNICAL FIELDS SEARCHED (IPC) |
| | | | G06V G06K |

The present search report has been drawn up for all claims

| Place of search | Date of completion of the search | Examiner |
|---|---|---|
| The Hague | 4 April 2022 | Peelen, Bastien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 20 7007

This annex lists the patent family members relating to the patent documents cited in the above-mentioned European search report.
The members are as contained in the European Patent Office EDP file on
The European Patent Office is in no way liable for these particulars which are merely given for the purpose of information.

04-04-2022

| Patent document cited in search report | | Publication date | Patent family member(s) | | Publication date |
|---|---|---|---|---|---|
| US 2020258223 | A1 | 13-08-2020 | US | 2020258223 A1 | 13-08-2020 |
| | | | US | 2021233238 A1 | 29-07-2021 |
| | | | US | 2021256690 A1 | 19-08-2021 |
| | | | US | 2022101519 A1 | 31-03-2022 |
| WO 2020083970 | A1 | 30-04-2020 | CN | 112912923 A | 04-06-2021 |
| | | | EP | 3871183 A1 | 01-09-2021 |
| | | | JP | 2022504634 A | 13-01-2022 |
| | | | US | 2021343009 A1 | 04-11-2021 |
| | | | WO | 2020083970 A1 | 30-04-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020200148939